# EUROPEAN PATENT APPLICATION

(11) **EP 4 596 711 A1**
(43) Date of publication of application: **06.08.2025**
(21) Application number: 23872648.3
(22) Date of filing: 25.09.2023
(51) Int. Cl.: C12Q 1/6806, C12N 15/11, C12Q 1/6869, C40B 40/06

(54) **DNA LIBRARY PREPARATION METHOD**

(30) Priority: 26.09.2022 JP 2022152384
(71) Applicant: Kyushu University, National University Corporation, Nishi-ku Fukuoka-shi Fukuoka 819-0395 (JP)
(72) Inventor: MIURA Fumihito, Fukuoka-shi, Fukuoka 819-0395 (JP)
(74) Representative: J A Kemp LLP
(86) International application number: PCT/JP2023/035820
(87) International publication number: WO 2024/071429

(57) **Abstract**

The present invention relates to a method for preparing a double-stranded DNA library from single-stranded DNA fragments and provides a highly efficient library preparation method, etc., that generates or contains few adapter dimers. The present invention is a method for preparing a double-stranded DNA library from single-stranded DNA fragments that includes the specific steps (i) to (v).

## Description

### TECHNICAL FIELD

The present invention relates to a method for preparing a double-stranded DNA library from single-stranded DNA fragments.

### BACKGROUND ART

Methods for efficiently preparing a double-stranded DNA library from single-stranded DNA fragments have been conventionally studied and developed. In particular, the library preparation method called "TACS-T4" developed by the present inventor (see Non-patent literature 1) is an excellent preparation method that is substantially 100 to 10,000 times more efficient in terms of library yield than existing methods, such as the method using CircLigase II (see Non-patent literature 2) or the method using T4 DNA ligase (see Non-patent literature 3). The above TACS-T4 uses "TACS ligation" technique (see Non-patent literature 4) previously developed by the present inventor. TACS (TdT-Assisted Chimeric ssDNA) ligation is a highly efficient single-stranded DNA ligation technique and is a unique reaction that ligates single-stranded DNAs to each other, which was developed based on the fact that the reaction efficiency is particularly high when the 3'-end of nucleotides on which the RNA ligase acts is RNA. TdT (Terminal Deoxynucleotidyl Transferase) can act efficiently on single-stranded DNA. This enzyme stops the elongation after adding 3-4 ribonucleotides to the 3'-end of single-stranded DNA in the presence of ribonucleotide triphosphate. TACS ligation is a technique that efficiently ligate single-stranded DNAs to each other by combining RNA tailing by the above TdT with the activity of RNA ligase.

While the library preparation method "TACS-T4" can improve the library yield as described above, there is a problem that a large amount of dimers (double-stranded adaptor dimers) of the adaptor sequences used for the preparation of a double-stranded library are produced as by-products in the reaction system along with the preparation of the double-stranded library. Production of such adaptor dimers causes problems in that a reagent is required to remove the dimers when the prepared library is read by a sequencer, and even if the dimers are to be removed by purification, if the difference in base length between the insert DNA to be sequenced and the adaptor sequence is small, even purification is difficult.

### CITATION LIST

### Non-patent Literature

Non-patent literature 1: Osamu Hisano et al, BMC Biology (2021) 19: 225
Non-patent literature 2: Marie-Theres Gansauge et al, Nat. Protoc., 2013 Apr; 8(4): 737-48
Non-patent literature 3: Marie-Theres Gansauge et al, Nucleic Acids Research, 2017, Vol. 45, No. 10 e79
Non-patent literature 4: Fumihito Miura et al, Nucleic Acids Research, 2019, Vol. 47, No. 15 e85

### SUMMARY OF INVENTION

Under these circumstances, with respect to the method for preparing a double-stranded DNA library from single-stranded DNA fragments, there has been a desire to develop a highly efficient library preparation method, etc. that produces or contains less adaptor dimer.

The present invention was made in consideration of the above circumstances and provides a method for preparing a double-stranded DNA library as follows.
[1] A method for preparing a double-stranded DNA library from single-stranded DNA fragments, comprising:
   (i) a step of ligating a single-stranded adaptor sequence A1 having a phosphorylated 5'-end to the 3'-end of a single-stranded DNA fragment having both ends dephosphorylated to produce adaptor-tagged single-stranded DNA;
   (ii) a step of annealing a single-stranded adaptor sequence A2 to the adaptor sequence A1 portion contained in the adaptor-tagged single-stranded DNA and then elongating the 3'-end of the adaptor sequence A2 by DNA polymerase to produce adaptor-tagged double-stranded DNA;
   (iii) a step of reacting topoisomerase with a double-stranded adaptor sequence B1, which has only one 5'-end phosphorylated and has a topoisomerase recognition sequence in the region containing the 3'-end complementary to said 5'-end, to produce a topoisomerase-linked double-stranded adaptor sequence B2 having topoisomerase conjugated to the 3'-end of said recognition sequence;
   (iv) a step of ligating the topoisomerase-linked double-stranded adaptor sequence B2 to the end of the adaptor-tagged double-stranded DNA, which end is tagged with no adaptor sequence, to produce double-stranded DNA having both ends tagged with adaptors; and
   (v) a step of performing PCR using the double-stranded DNA having both ends tagged with adaptor sequences as a template to obtain a library containing a plurality of said double-stranded DNAs.
[2] The method according to [1] above, wherein the topoisomerase-linked double-stranded adaptor sequence B2 is not ligated to a double-stranded adaptor sequence C produced by annealing between unreacted single-stranded adaptor sequence A1 and unreacted single-stranded adaptor sequence A2.
[3] The method according to [1] above, wherein:
   the double-stranded adaptor sequence B1 has a nick in the region containing the phosphorylated 5'-end; and
   the single-stranded adaptor sequence A1 has uracil or dSpacer in the sequence.
[4] The method according to [3] above, wherein the step (iv) comprises adding endonuclease to the reaction system after the production.
[5] The method according to [1] above, wherein the ligation and production in step (i) are performed using the TACS ligation scheme.
[6] A double-stranded DNA library obtained by the method according to any one of [1]-[5] above.

### ADVANTAGEOUS EFFECTS OF INVENTION

The present invention provides a method for preparing a double-stranded DNA library from single-stranded DNA fragments, which can increase the library yield extremely efficiently compared to conventional double-stranded DNA library preparation methods (Non-patent literature 2 and 3 above) and which can even effectively suppress the production of double-stranded adaptor dimers as by-products during the preparation.

### BRIEF DESCRIPTION OF DRAWINGS

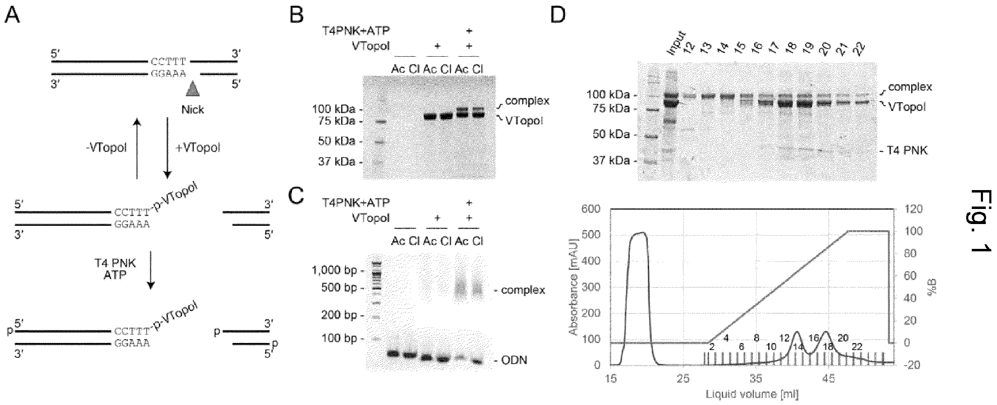

Figure 1: Figure showing the results of VTopoI-oligonucleotide complex (VOC) formation and purification (VTopoI: topoisomerase I from vaccinia virus).
   A. Scheme of complex formation. VOC formation is an equilibrium reaction. Therefore, phosphorylation of released DNA is an effective way to increase the amount of the complex.
   B and C. Examples of VOC formation. Addition of T4 PNK increases complex formation. Images from SDS-PAGE (B) and agarose gel electrophoresis using E-Gel Ex (Thermo Fisher Scientific) (C).
   D. Purification of VOC by heparin column chromatography. Results of fractional analysis by SDS-PAGE (top) and chromatogram (bottom). VOCs are found to elute faster than VTopoI.

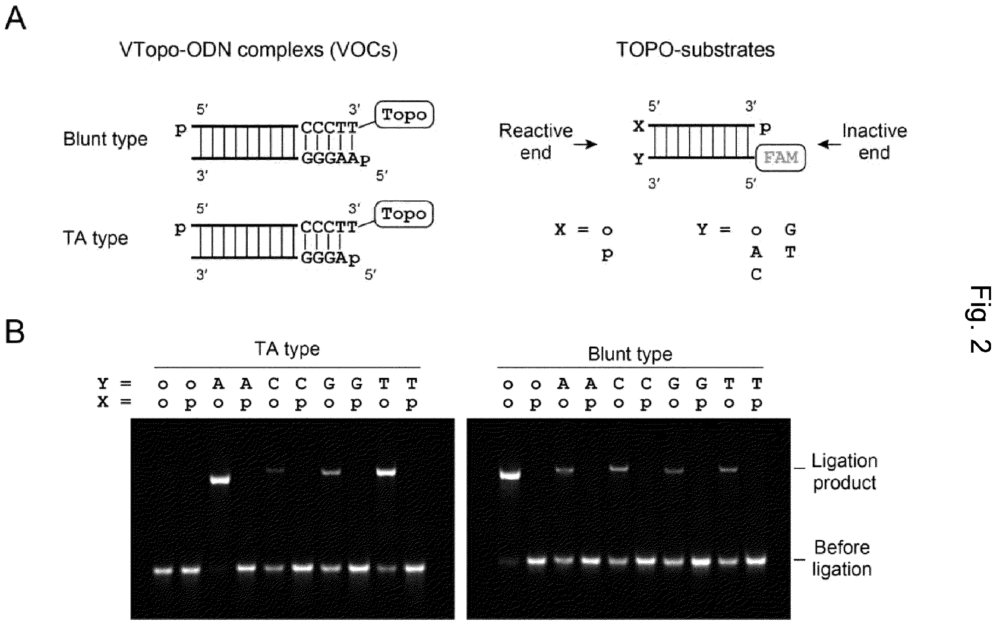

Figure 2: Figure showing the results of the study of substrate specificity of VOCs.
   A. Structures of VOCs (left) and TOPO-substrates (right).
   B. Gel electrophoresis images after mixing VOCs and TOPO-substrates. TA (left) and blunt (right) type VOCs were examined for reactivity with substrates in all combinations of 5'-phosphate and single-base overhang. If ligation occurs, the band is expected to move to the upper side.
   The symbols have the following meanings: o: hydroxyl end, p: phosphorylated end, A, C, G, or T: nucleotide bound to the 5'-end of the substrate. Details of the experiments are as follows.
   <Studies on the specificity of ligation of TOPO-activated adaptors>
   0.5× TACS buffer, 1× ExTaq buffer, 200 µM ATP, 200 µM each dNTP, and 20% (w/v) PEG6000 were incubated at 95°C for 3 minutes and 55°C for 5 minutes. After lowering the reaction temperature to or below 37°C, 5 µL of 0.5 M EDTA solution (pH 8.0) and 150 ng of VOC were added and incubated at room temperature for 15 minutes. Next, 45 µL of buffer B2 and 5 µL of proteinase K were added to the reaction solution and incubated at 50°C for 15 minutes. 100 µL of isopropanol was added to the reaction solution and the mixture was loaded into ZymoSpin column (Zymo Research). The column was washed with Buffer PE (Qiagen) and DNA was eluted with 10 µL of 10 mM Tris-HCl, pH 8.5. The purified DNA was loaded onto a 10% acrylamide gel (TBE buffer system). After electrophoresis, gel images were taken with ChemiDock imaging system.

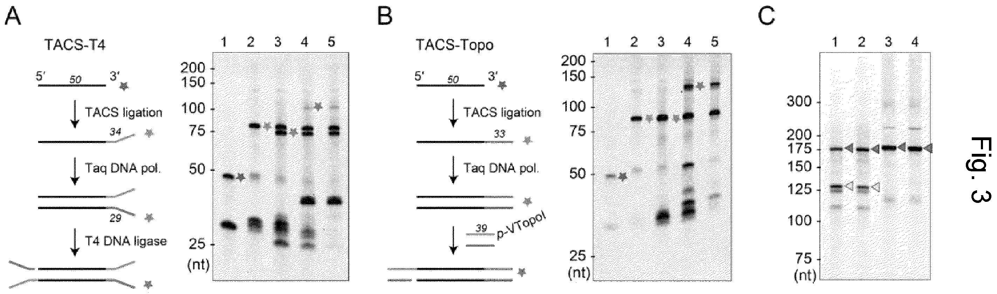

Figure 3: Figure showing the results of comparisons of schemes for preparing a library from single-stranded DNA.
   A and B. TACS-T4 (A) and TACS-TOPO (B). Schemes (left) and the results of DNA analysis at each reaction step using denaturing gel electrophoresis (right) are shown. Numbers in italic font indicate the lengths of DNAs used. The colored stars in the left schemes correspond to the bands in the right gel images. The lanes each refer to the following; 1: before TACS ligation, 2: after TACS ligation, 3: after synthesis of complementary strand by Taq DNA polymerase, 4: after second adaptor ligation, and 5: after SPRI purification.
   C. Denaturing gel electrophoresis image of the libraries after PCR amplification. DNA fragments amplified from the libraries prepared by TACS-T4 (lanes 1 and 2) and TACS-TOPO (lanes 3 and 4) were loaded onto denaturing gels. The appearance of adaptor dimers was seen only in the libraries prepared by TACS-T4. Libraries containing inserts are indicated by red arrowheads and dimers by yellow arrowheads.

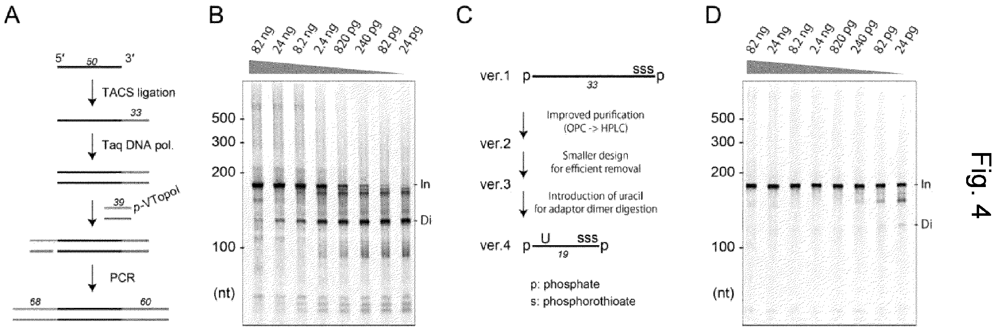

Figure 4: Figure showing the improvements to the TACS-TOPO scheme and the results thereof.
   A and B. In the first version (ver. 1) of the TACS-TOPO scheme (A), production of adaptor dimers was observed (B).
   C and D. Several modifications to the adaptor used in TACS ligation (C) improved the sensitivity of dimer-less library preparation and enabled preparation of highly sensitive library from ssDNA (D). In D, ver. 4 adaptor is used.

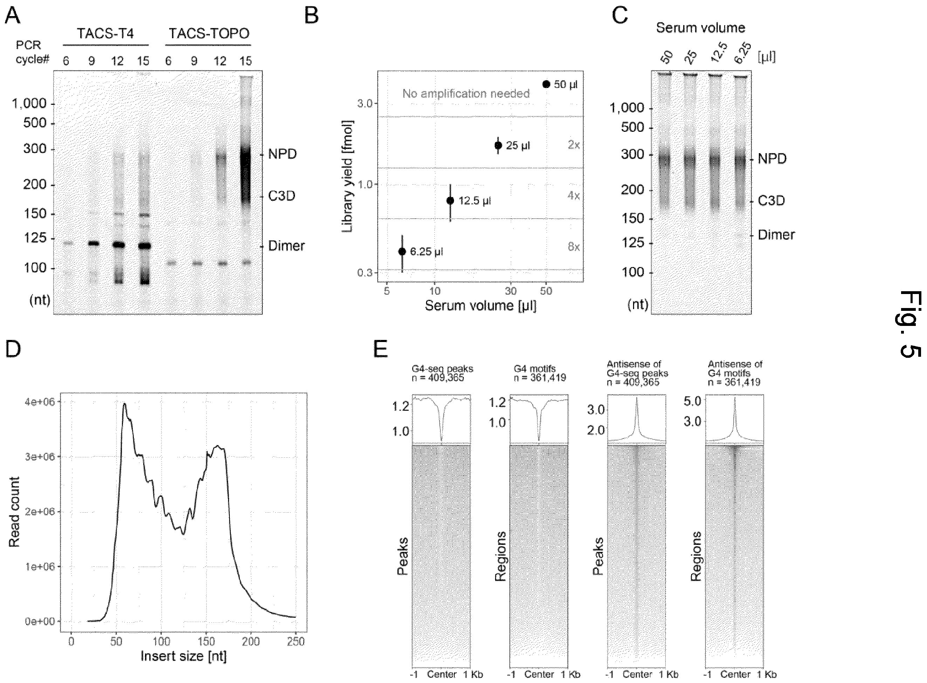

Figure 5: Figure showing the results of the preparation of sequencing libraries from cell-free DNA (cfDNA).
   A. Comparison of libraries prepared by TACS-T4 and TACS-TOPO. NPD: nucleosome-protected DNA fragment, C3D: short cell-free single-stranded DNA.
   B. Library yields using TACS-TOPO ver 4. Library yields from various volumes of serum are shown. The zones separated by horizontal lines indicate the magnitude of amplification of the library required to obtain 90 Gb reads.
   C. Examples of electrophoretic analysis of sequencing libraries prepared from cfDNA using TACS-TOPO ver. 4. After electrophoresis on a 6% denaturing gel, the gel was stained with SYBR Gold and an image of the gel was taken.
   D. Size distribution of the reads mapped to the human reference genome. An example of a library prepared from 50 µL of serum using TACS-TOPO ver 4. After sequencing, the library was mapped to the human reference genome.
   E. Peaks identified by G4-seq and G4 motifs co-localized with the reads. Fragments of 35-75 nucleotides were selected from the mapped reads and analyzed for co-localization with the G4-seq peaks and G4 motifs.

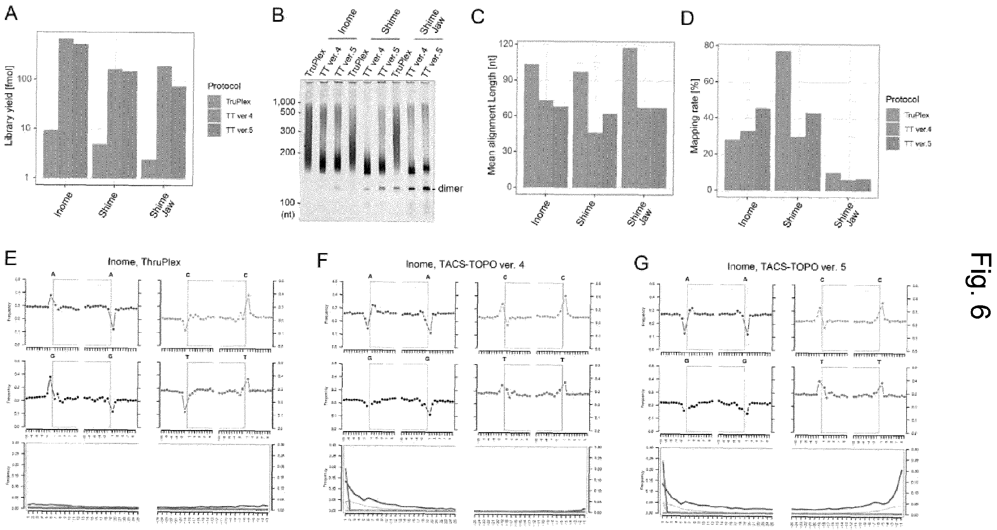

Figure 6: Figure showing the results of comparison of library preparation by the TACS-TOPO scheme using DNA from ancient human bones and a conventional protocol adapted only for dsDNA.
   A-D. Library yields when the whole libraries were amplified by 12 cycles of PCR (A), size distributions of the libraries (gel electrophoresis analysis, B), mean alignment lengths of the sequenced reads (C), and mean mapping rates of the reads to the human reference genome (D) are shown. ThruPlex DNA-seq kit was selected as the representative protocol because it demonstrated the best library preparation sensitivity among commercially available library preparation kits.
   Libraries prepared with ThruPlex (E), TACS-TOPO ver. 4 (F), and TACS-TOPO ver. 5 (G) were sequenced and mapped to the reference genome. Patterns of mismatch-detected sites when the results were analyzed using mapDamage are shown.

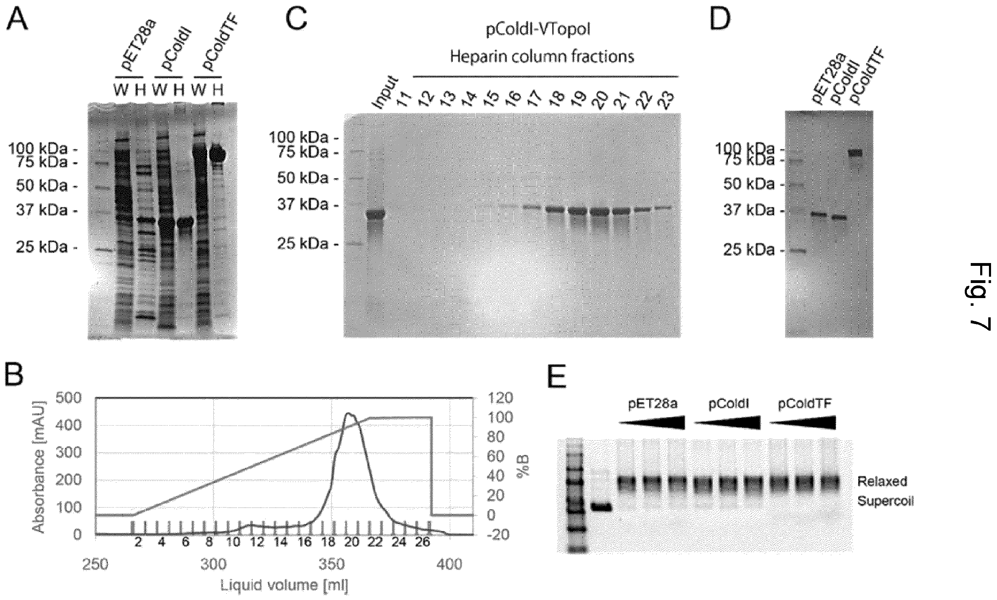

Figure 7: Figure showing the results of purification of VTopoI.
   A. Results of SDS-PAGE analysis of VTopoI expressions with three different expression vectors.
   B and C. Heparin column purification of VTopoI. An example of purification of VTopoI prepared with pColdI. Chromatogram (B) and SDS-PAGE gel image (C) are shown.
   D. SDS-PAGE gel image of purified VTopoI.
   E. Supercoil-relaxation assay of purified VTopoI. Details of the assay are as follows
   <Supercoil-relaxation assay (topoisomerase activity assay)>
   To 20 µL of a reaction solution containing 180 ng (100 fmol) of pUC19 DNA in 50 mM Tris-HCl, pH 8.0, and 75 mM NaCl, either 0, 10, 20, or 40 pmol of VTopoI was added and incubated at room temperature for 10 minutes. Next, 2 µL of 10% (w/v) sodium dodecyl sulfate (SDS) was added to the reaction solution and loaded onto a 1% (w/v) agarose gel (TAE buffer system). After electrophoresis, the gel was stained with SYBR Gold gel stain (Thermo Fisher Scientific) and an gel image was taken with ChemiDock imaging system (Bio-rad Laboratories, Hercules, CA).

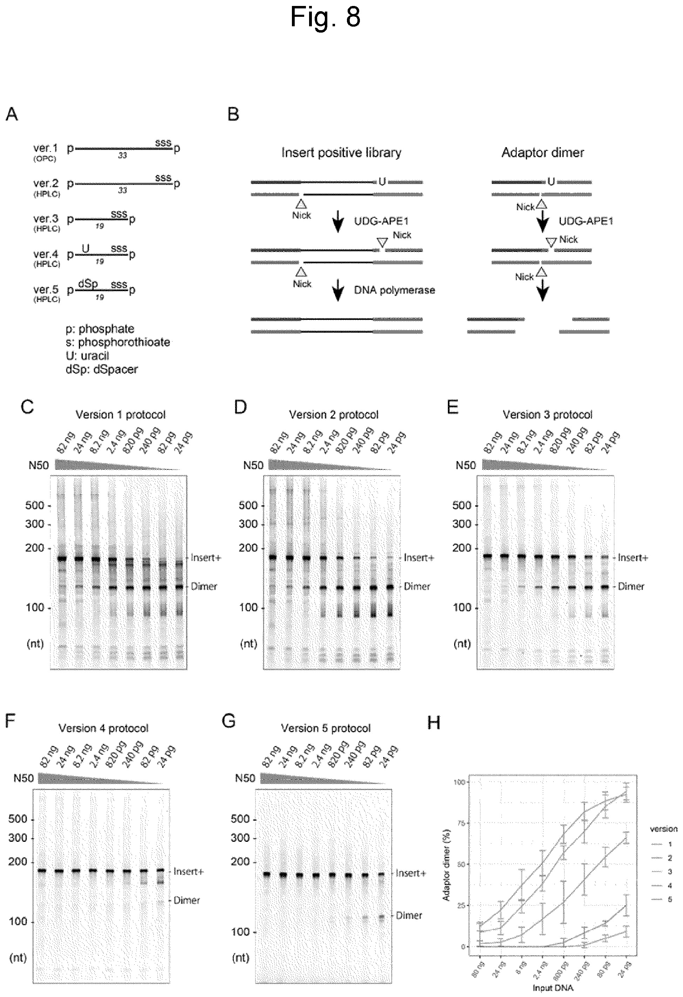

Figure 8: Figure showing modifications to adaptors for preparation of an adaptor dimer-free library and the results thereof.
   A. Structures of the adaptors studied.
   B. Principle of uracil-containing adaptor that suppresses adaptor dimer formation.
   C-H. Studies of TACS-TOPO protocols and the amounts of dimers formed. Representative gel images of the libraries after amplification by PCR (C-G) and a summary of the relative amounts of dimers formed (H). Libraries were prepared from various amounts of chemically synthesized DNA of random 50 nucleotides (N50), amplified by PCR, and analyzed by denaturing gel electrophoresis. It can be seen that the relative amount of the adaptor dimers has decreased due to the modifications to the adaptor. In order to draw graph H, three independent experiments were carried out.

### DESCRIPTION OF EMBODIMENTS

Hereinafter, the present invention will be described in detail. The scope of the present invention is not limited to these descriptions, and the present invention may be implemented with modifications other than those illustrated in the following examples without departing from the spirit of the invention. Note that this specification incorporates the entirety of Japanese Patent Application No. 2022-152384 (filed on September 26, 2022), upon which priority is claimed in this application.

All publications cited herein, such as prior art literature, as well as published patent applications, patent publications, and other patent literature, are incorporated herein by reference.

### 1. Summary of the Invention

The present invention provides a highly efficient technique for preparing a sequencing library from single-stranded DNA (ssDNA). For the initial adaptor ligation to single-stranded DNA, TACS ligation, which was recently developed by the present inventor, is used. Then, after synthesizing a complementary strand to the adaptor-tagged single-stranded DNA, second adaptor ligation is performed by adaptor ligation using vaccinia virus topoisomerase I (VTopoI). Based on these technical principles, the TACS-TOPO scheme is capable of preparing a sequencing library with less adaptor dimer production and lower adaptor dimer content from as little as 24 pg of single-stranded DNA. The TACS-TOPO scheme was capable of preparing a library for cell-free DNA (cfDNA) analysis from 50 µL of human serum without amplification. In addition, a protocol based on the TACS-TOPO scheme was used in an attempt to analyze DNA extracted from ancient human bones. As a result, the library yield was improved by two orders of magnitude compared to a conventional library preparation method.

### 2. Method for preparing DNA library

A method for preparing a DNA library according to the present invention (hereinafter also referred to as "the library preparation method of the present invention") is a method for preparing a double-stranded DNA library from single-stranded DNA fragments.

Specifically, the library preparation method of the present invention comprises the following steps (i)-(v), but is not limited to these steps and may comprise any other steps.
(i) A step of ligating a single-stranded adaptor sequence A1 having a phosphorylated 5'-end to the 3'-end of a single-stranded DNA fragment having both ends dephosphorylated to produce adaptor-tagged single-stranded DNA (step (i));
(ii) a step of annealing a single-stranded adaptor sequence A2 to the adaptor sequence A1 portion contained in the adaptor-tagged single-stranded DNA and then elongating the 3'-end of the adaptor sequence A2 by DNA polymerase to produce adaptor-tagged double-stranded DNA (step (ii));
(iii) a step of reacting topoisomerase with a double-stranded adaptor sequence B, which has only one 5'-end phosphorylated and has a topoisomerase recognition sequence in the region containing the 3'-end complementary to said 5'-end, to produce a topoisomerase-linked double-stranded adaptor sequence B having topoisomerase conjugated to the 3'-end of said recognition sequence (step (iii));
(iv) a step of ligating the topoisomerase-linked double-stranded adaptor sequence B to the end of the adaptor-tagged double-stranded DNA, which end is tagged with no adaptor sequence, to produce double-stranded DNA having both ends tagged with adaptor sequences (step (iv)); and
(v) a step of performing PCR using the double-stranded DNA having both ends tagged with adaptor sequences as a template to obtain a library containing a plurality of said double-stranded DNAs (step (v)).

### -Regarding step (i)

The "single-stranded DNA fragment" used as the so-called insert DNA in the library preparation is one that is dephosphorylated at both ends (5'-end and 3'-end). Note that the single-stranded DNA fragment preferably has ribonucleotides added to its 3'-end and that the ribonucleotides are preferably made up of a few bases, more preferably 1-3 bases, or 3 bases (e.g., adenines (rA) as three ribonucleotides). Addition of the ribonucleotides, i.e., RNA-tailing (or ribo-tailing), is preferably done using, for example, TdT (Terminal Deoxynucleotidyl Transferase). TdT stops elongation by adding ribonucleotides to the 3'-end of the single-stranded DNA in the presence of ribonucleotide triphosphate. Next, "single-stranded adaptor sequence A1", i.e., single-stranded DNA whose 5'-end is phosphorylated, is ligated to the 3'-end of the single-stranded DNA fragment obtained above. This ligation is preferably done using, for example, an RNA ligase. Through this ligation, "adaptor-tagged single-stranded DNA" is produced. For the details of how the adaptor-tagged single-stranded DNA is produced in step (i) (including the conditions of the various reactions, etc.), reference can be made, for example, but not limited to, the "TACS ligation" technique disclosed in Non-patent literature 4 (Nucleic Acids Research, 2019) mentioned above as appropriate. If there is an enzyme with higher ligation activity and/or efficiency than that of the enzyme used in the TACS ligation, it can be used as an alternative as appropriate.

### -Regarding step (ii)

This is a step of making the adaptor-tagged single-stranded DNA produced in step (i) into double-stranded DNA. In detail, "single-stranded adaptor sequence A2", which is single-stranded DNA, is annealed to the adaptor sequence A1 portion in the produced adaptor-tagged single-stranded DNA. The single-stranded adaptor sequence A2 (especially its 3'-end portion) preferably has a nucleotide sequence that is complementary to at least a part of the nucleotide sequence of the adaptor sequence A1 portion. After the annealing, the 3'-end of the adaptor sequence A2 is elongated by DNA polymerase. The DNA polymerase is preferably, but not limited to, Taq DNA polymerase, etc. Thus, the resulting double-stranded DNA, "adaptor-tagged double-stranded DNA", is produced. For details of how the adaptor-tagged double-stranded DNA is produced in step (ii) (including the conditions of the various reactions, etc.), reference may be made to some of the steps in the "TACS-T4" library preparation method disclosed in Non-patent literature 1 (BMC Biology, 2021) mentioned above as appropriate.

### -Regarding step (iii)

This is a step of preparing "double-stranded adaptor sequence B1", which is an adaptor sequence of double-stranded DNA for the so-called second ligation (specifically, step (iv) below). First, the double-stranded adaptor sequence B1 has the 5'-end of one single-stranded DNA phosphorylated, but not the 5'-end of the other single-stranded DNA, and has a topoisomerase recognition sequence in the region containing the 3'-end that is complementary to the phosphorylated 5'-end. This topoisomerase is preferably topoisomerase I, and more preferably topoisomerase I from vaccinia virus (VTopoI). Examples of the VTopoI recognition sequence include, but are not limited to, 5'-CCTTT-3'- and 5'-(C/T)CCTT-3'. VTopoI specifically recognizes sequences consisting of these five nucleotides and forms a covalent bond with the phosphate group of 3'-T thereof. Along with the above formation, the nucleotide sequence on the 3' side of the above 3'-T of the recognition sequence is cleaved. It is preferred that the region including the phosphorylated 5'-end of the double-stranded adaptor sequence B1 has a nick (break in the sequence), which is preferably located, for example, but not limited to, at a nucleotide on the 5' side of the sequence complementary to the VTopoI recognition sequence, and more preferably one nucleotide 5' to said complementary sequence. With this nick, when the nucleotide sequence on the 3' side of the above 3'-T of the recognition sequence is cleaved, the sequence containing the sequence complementary to the cleaved sequence is also cleaved from the double-stranded adaptor sequence B1. In this manner, "topoisomerase-linked double-stranded adaptor sequence B2" is produced. If the double-stranded adaptor sequence B1 has the aforementioned nick, the nick or the deleted portion corresponding to the nick preferably remains in the double-stranded adaptor sequence B2.

### -Regarding step (iv)

The topoisomerase-linked double-stranded adaptor sequence B2 produced in step (iii) is ligated to the end of the adaptor-tagged double-stranded DNA produced in step (ii), which end is tagged with no adaptor sequence. Topoisomerase (VTopoI) has the activity of ligating the phosphate group of 3'-T (i.e., the phosphorylated 3'-end), to which topoisomerase itself is covalently bound, to the dephosphorylated 5'-end of the adaptor-tagged double-stranded DNA (i.e., the hydroxyl group at the 5'-end). As a result, "double-stranded DNA having both ends tagged with adaptors" is produced. On the other hand, the topoisomerase-linked double-stranded adaptor sequence B2 does not, in principle, ligate to an unreacted double-stranded adaptor (with both strands phosphorylated at the 5'-ends) in which the aforementioned single-stranded adaptor sequence A1 and single-stranded adaptor sequence A2 are annealed (i.e., free sequence A1 and sequence A2 are annealed). Therefore, formation of a dimer between the unreacted adaptor and the adaptor sequence B2 is effectively suppressed. Even if such a dimer is formed accidentally, for example, if the double-stranded adaptor sequence B1 has a nick as described in step (iii) above (note that the nick or the deleted portion corresponding to the nick remains in the double-stranded adaptor sequence B2), and if the single-stranded adaptor sequence A1 has uracil in its sequence, the dimer will be degraded by adding DNA endonuclease to the reaction system after the production of the "double-stranded DNA with both ends tagged with adaptors", resulting in a state as if no dimer had been formed. Examples of this DNA endonuclease include those having the activity of hydrolyzing the N-glycosidic bond of uracil or forming a nick in the backbone of DNA on the 5' side of uracil. For example, UDG (uracil-DNA glycosylase), APE 1 (human apurinic/apyrimidinic (AP) endonuclease), EndoQ (endonuclease Q), etc. are preferred. Removal of uracil or nicking in the vicinity of uracil by DNA endonuclease and the nick in the double-stranded adaptor sequence B2 (more specifically, the nick at the site of ligation to the double-stranded adaptor sequence B2) will cause the formed adaptor dimer to degrade (split) and consequently be removed from the reaction system. In addition, "dSpacer" as a spacer-modified DNA oligo can be introduced in place of uracil in the aforementioned single-stranded adaptor sequence A1. A spacer-modified DNA oligo is a base-free structure that can be inserted into the phosphodiester bond backbone of the DNA strand. There are types that lack only the base or that lack both the base and the deoxyribose ring, and the number of the spacer arms can be selected variably. dSpacer lacks the base and can destabilize the double strand of the inserted sequence portion, and can also control the stability of the double strand depending on the incorporated site and structure. When incorporated as described above, dSpacer has the effect of preventing the formation of adaptor dimers and also allows for easy removal of adaptor dimers by enzymatic degradation. Note that instead of or in combination with dSpacer, "ethynyl-dSpacer" (which has an alkyne structure attached to the 1' position of the sugar to which the base is attached in a normal oligo-DNA strand) can also be employed.

### -Regarding step (v)

This is a step of performing PCR using the produced double-stranded DNA having both ends tagged with adaptor sequences as a template to obtain a library containing a plurality of said double-stranded DNAs. The reaction conditions of PCR, the purification conditions of the obtained PCR products (library), etc. can be set as desired or reference may be made to some of the steps in the "TACS-T4" library preparation method disclosed in Non-patent literature 1 (BMC Biology, 2021) mentioned above as appropriate.

According to the present invention, a double-stranded DNA library obtained by the library preparation method of the present invention described above is also included in the scope of the present invention.

Hereinafter, the present invention will be described more specifically by means of examples, although the present invention should not be limited thereto.

### [Example 1]

### 1. Summary

There are many methods for preparing a sequencing library from double-stranded DNA (dsDNA), but methods for single-stranded DNA (ssDNA) have limited number of options. There are three main principles for preparing a sequencing library from single-stranded DNA.

The first approach is to add a homopolymer to the 3'-end of single-stranded DNA by terminal deoxynucleotidyl transferase (TdT). After the homopolymer addition, this tail is used as a primer binding site for converting the single-stranded DNA into double-stranded DNA, which is subsequently subjected to second adaptor ligation by T4 DNA ligase. Library preparation using TdT is highly efficient, and library preparation kits based on this principle are commercially available, but the presence of long terminal tails may be an obstacle in subsequent sequencing and analysis.

The second method is based on single-stranded DNA ligation reaction by RNA ligase, where a 5'-phosphorylated adaptor is ligated to the 3'-end of the single-stranded DNA. For example, Gansauge et al. have established a method based on CircLigase II [References 1 and 2]. After adaptor ligation, conversion of the single-stranded DNA into double-stranded DNA and second adaptor ligation using T4 DNA ligase follow. Although RNA ligase can ligate two single-stranded DNAs to each other, its reaction efficiency is less than a few percent. Therefore, the yield of a library preparation method using RNA ligase is extremely limited.

The third approach is a method using T4 DNA ligase for single-stranded DNA ligation [Reference 3]. In this method, a double-stranded adaptor with a single-stranded random hexamer at one end is used. If the random hexamer can attach perfectly to the end of the target single-stranded DNA, the nick at the end of the partially double-stranded adaptor will be ligated by T4 DNA ligase. This splint ligation-based method is called ssDNA2.0 and has been shown to be more efficient than the method using CircLigase II [Reference 3]. However, while both RNA ligase- and T4 DNA ligase-based methods have the advantage of not introducing an extra nucleotide insert at the ligation site, the efficiency of library preparation is low and there is room for improvement.

In order to improve the efficiency of preparing a sequencing library from single-stranded DNA, the present inventor developed an adaptor ligation scheme for single-stranded DNA named TACS ligation [Reference 4]. TACS ligation involves the addition of a few adenylates to single-stranded DNA by TdT, which is called ribo-tailing by the present inventor. An adaptor is then ligated to the ribo-tailed single-stranded DNA by RNA ligase. Ribo-tailing the 3'-end of single-stranded DNA can greatly improve the ligation efficiency of RNA ligase by making the 3'-end of the target single-stranded DNA look like RNA. As a result, TACS ligation can ligate the adaptor to the 3'-end of 80% or more of the target single-stranded DNA [Reference 4]. It is worth noting that the ribonucleotide tailing reaction by TdT automatically stops after 1-3-base long nucleotide elongation [Reference 5], so the ribo-tail itself has little effect on the subsequent sequencing. TACS ligation has been used for the purpose of analyzing short single-stranded DNA contained in cell-free DNA in human blood and has been shown to be effective. After ligating the adaptor to cell-free DNA (cfDNA) by the TACS ligation scheme, the target single-stranded DNA is converted into double-stranded DNA, which was then subjected to the second adaptor tagging reaction by T4 DNA ligase (TACS-T4 scheme). The TACS-T4 scheme resulted in a library yield that was improved by several orders of magnitude compared to methods using CircLibase II or T4 DNA ligase [Reference 6]. However, although the TACS-T4 scheme improved the library yield, there were practical problems to be solved. Specifically, the inefficiency of the second adaptor ligation reaction and the production of non-negligible amount of adaptor dimers. Ligation efficiency can be improved by optimizing the reaction, but this will simultaneously increase the amount of adaptor dimers formed. If the unreacted adaptors are removed before the second adaptor ligation reaction, the formation of adaptor dimers can be avoided. However, the additional purification step will inevitably result in the loss of scarce DNA. Furthermore, when targeting relatively small DNA molecules, the removal of unreacted adaptors is quite difficult because there is no difference in size between the adaptors and the library molecules of interest. Therefore, the present inventor thought it was necessary to develop an alternative method with less adaptor dimer formation and higher adaptor ligation efficiency. For this purpose, the present inventor thought that an adaptor ligation reaction using vaccinia virus topoisomerase (VTopoI), which has a different reaction direction from that of T4 DNA ligase, might be more efficient. In other words, while many DNA/RNA ligases ligate DNA having a phosphorylated 5'-end to the hydroxyl group at the 3'-end of the target DNA, VTopoI ligates DNA having a phosphorylated 3'-end to the hydroxyl group at the 5'-end of the target DNA. Due to this difference in substrate specificity, VTopoI would in principle show reactivity with the adaptor used in the initial adaptor ligation, which has its 5'-end phosphorylated.

The gene encoding VTopoI was identified by Stewart Shuman in 1987 [Reference 7]. Since then, he and his colleagues have investigated the properties of this enzyme in detail [References 8-16]. The idea of using VTopoI as a tool for gene cloning was first described in 1994 [Reference 17]. This technology was subsequently commercialized by Invitrogen (now Thermo Fisher Scientifics). Since then, VTopoI has been used as an efficient enzyme for incorporating a DNA fragment into a vector by the so-called TOPO cloning technique [Reference 18]. Once activated by VTopoI, the vector DNA is ready to be ligated to the target DNA. This reaction is immediate, highly efficient and specific. Because of these properties, TOPO cloning has been one of the options frequently employed as the first step to initiate gene cloning. On the other hand, despite the fact that the properties of VTopoI are very attractive for the development of a new technique, there is a very limited number of examples of applications of ligation by VTopoI in references [Reference 19].

In this example, VTopoI was used in combination with TACS ligation for the purpose of preparing a sequencing library from single-stranded DNA in a highly efficient manner. Preparation of VTopoI, formation and purification of a set of VTopoI and oligonucleotide (ODN) complexes (VOCs), establishment of the TACS-TOPO scheme adapted for the preparation of a library from single-stranded DNA of VOCs, and its application will be described.

### 2. Materials and methods

### -Vector construction

The gene encoding VTopoI (GenBank accession number: L13447.1) was optimized for the codon usage frequency in *E. coli* strain K-12 and artificially synthesized by Eurofins Genomics without internal BamHI and EcoRI recognition sequences, but with BamHI and EcoRI recognition sequences at the 5'- and 3'-ends of the gene, respectively (SEQ ID NO: 27).

### [Chem 1]

### Artificial synthetic gene encoding VTopol (BamHI and EcoRI sites are underlined)

GGATCCATGCGTGCATTGTTCTACAAAGACGGGAAACTTTTTACCGACAATAATTTCCTG
AATCCGGTAAGTGACGACAATCCAGCGTATGAAGTTTTGCAGCACGTGAAAATTCCGACC
CATCTTACCGATGTTGTGGTTTATGAACAAACGTGGGAAGAAGCTCTGACCCGTCTGATT
TTCGTTGGCAGTGATTCAAAAGGTCGAAGACAGTACTTCTACGGGAAAATGCATGTCCAG
AATCGCAACGCAAAACGGGATCGCATATTTGTGCGCGTATATAACGTGATGAAGCGCATC
AATTGTTTCATCAACAAAAACATCAAAAAGAGCTCAACTGATTCGAACTATCAGTTAGCC
GTGTTTATGCTCATGGAGACCATGTTCTTCATTCGCTTTGGCAAAATGAAGTACCTCAAA
GAGAATGAAACGGTAGGCTTACTGACCTTAAAGAACAAACACATTGAAATCAGCCCTGAT
GAGATTGTGATCAAATTTGTGGGTAAAGACAAAGTCAGCCATGAATTTGTCGTCCACAAA
TCCAATCGCCTGTATAAACCGCTGCTGAAACTCACTGACGATTCTTCCCCAGAAGAATTT
CTGTTTAACAAACTGTCTGAACGTAAGGTGTACGAGTGCATTAAGCAATTCGGTATTCGT
ATTAAGGATCTGCGTACGTATGGTGTCAACTATACGTTTCTGTACAACTTTTGGACCAAC
GTAAAGAGCATATCTCCCTTACCTAGTCCGAAAAAACTGATTGCGCTTACCATCAAACAG
ACAGCGGAAGTTGTTGGACATACACCGAGCATCTCAAAACGTGCCTATATGGCTACGACT
ATCCTAGAAATGGTGAAAGACAAAAATTTCCTGGATGTTGTTTCGAAAACGACCTTTGAT
GAGTTTTTGTCGATTGTCGTGGATCATGTGAAAAGCTCCACAGATGGCGAATTC

(SEQ ID NO: 27)

DNA fragments cleaved with BamHI and EcoRI were subcloned into BamHI-EcoRI sites of pColdTF, pColdI, and pET28a. In order to produce T4 polynucleotide kinase (T4 PNK), the pseT gene of T4 phage was amplified by PCR from genomic DNA (T4 GT7 DNA, Nippon Gene Co., Ltd.) with BamHI and EcoRI recognition sequences at the 5'- and 3'-ends, respectively (SEQ ID NO: 28) and cloned into the BamHI-EcoRI site of pColdI.

### [Chem 2]

### PCR-amplified gene encoding T4 polynucleotide kinase cloned into pColdI (BamHI and EcoRI sites are underlined; primer sequences for gene amplification are in italics)

ATGGGTCGCGGATCCATGAAAAAGATTATTTTGACTATTGGCTGTCCTGGTTCTGGTAAG
AGTACTTGGGCTCGTGAATTTATTGCTAAGAATCCCGGGTTTTATAATATCAATCGTGAT
GACTATCGCCAATCTATTATGGCGCATGAAGAACGCGATGAGTACAAGTATACCAAAAAG
AAAGAAGGTATCGTAACTGGTATGCAGTTTGATACAGCTAAAAGTATTCTGTACGGTGGC
GATTCTGTTAAGGGAGTAATCATTTCAGATACTAACCTGAATCCTGAACGTCGCCTAGCA
TGGGAAACTTTTGCCAAAGAATACGGCTGGAAAGTTGAACATAAAGTGTTTGATGTTCCT
TGGACTGAATTGGTTAAACGTAACTCAAAACGCGGAACTAAAGCAGTACCAATTGATGTT
TTACGTTCAATGTATAAAAGCATGCGAGAGTATCTCGGTCTTCCAGTATATAATGGGACT
CCTGGTAAACCAAAAGCAGTTATTTTTGATGTTGATGGTACACTAGCTAAAATGAATGGT
CGTGGTCCTTATGACCTTGAAAAATGCGATACCGATGTTATCAATCCTATGGTTGTTGAA
CTGTCTAAGATGTATGCTCTTATGGGTTATCAAATCGTAGTCGTTTCAGGTCGTGAAAGT
GGAACTAAAGAAGACCCAACGAAATATTATCGTATGACCCGTAAATGGGTTGAGGACATT
GCTGGCGTTCCATTAGTTATGCAATGTCAGCGCGAACAAGGCGATACCCGTAAAGACGAT
GTAGTTAAAGAAGAAATTTTCTGGAAACACATTGCACCGCATTTTGACGTGAAATTAGCT
ATTGATGACCGAACTCAAGTAGTTGAAATGTGGCGTCGTATCGGTGTTGAATGCTGGCAA
GTCGCTTCGGGAGATTTTGAATTCGAGCTCCGT
(SEQ ID NO: 28)

These expression vectors were introduced into T7Express (New England Biolabs, Ipswitch, MA) and the *E. coli* transformant was grown in LB medium (1% (w/v) tryptone, 0.5% (w/v) yeast extract and 0.5% (w/v) sodium chloride) containing 100 µg/ml carbenicillin (for pColdTF and pColdI, Nacalai Tesque, Inc.) or 50 µg/ml kanamycin (for pET28a, Wako Pure Chemicals).

### -Purification of enzyme

### Culture of bacteria

*E. coli* transformed with the expression vector was inoculated into an LB medium containing 3 ml of antibiotic and incubated at 37°C overnight with shaking. The seed culture was transferred into a 2 L Erlenmeyer flask containing 1 L of antibiotic-containing LB medium and further incubated at 37°C with shaking. Then, for pColdI and pColdTF, the flasks were cooled in ice water for 30 min and protein expression was induced by adding 238 mg of isopropyl-β-D-thiogalactopyranoside (IPTG). For pET28a, expression induction was initiated by adding 238 mg of IPTG without cooling. These flasks were continuously subjected to shaking culture at 37°C for 4 hours for pET28a and at 16°C overnight for pColdI and pColdTF. Cells were centrifuged at 2,500 × g for 15 minutes and stored at -80°C until immediately before use.

### Purification of recombinant proteins

The collected *E. coli* cells were suspended in HisTrap buffer A (20 mM sodium phosphate, pH 7.5, 800 mM sodium chloride, 20 mM imidazole, 1 mM dithiothreitol (DTT), and 10% (v/v) glycerol) containing a protease inhibitor reaction mixture (Nacalai Tesque, Inc.), and disrupted by an ultrasonic disruptor. After centrifuging this suspension at 15,000 × g for 15 minutes, the supernatant was filtered through a 0.45 µm filter syringe to make a cleared lysate. First-stage purification by chromatography was performed using HisTrap buffer A and HisTrap buffer B (20 mM sodium phosphate, pH 7.5, 800 mM sodium chloride, 200 mM imidazole, 1 mM dithiothreitol (DTT), and 10% (v/v) glycerol) and a 5 mL HisTrap HP column (Cytiva, Marlborough, MA). Next, the fraction containing the target protein was diluted 10-fold with heparin buffer A (50 mM sodium phosphate, pH 7.5, 100 mM sodium chloride) and further fractionated using heparin buffer A and heparin buffer B (50 mM sodium phosphate, pH 7.5, 1 M sodium chloride) and 5 mL of Hi Trap Heparin HP (Cytiva). The fraction containing the target protein was then collected and buffer-exchanged using a HiPrep 26/10 Desalting column with a preservation buffer (50 mM Tris-HCl, pH 8.0, 300 mM sodium chloride). These purifications by chromatography were performed using AKTA start (Cytiva). In AKTA start, purification was performed using default affinity, ion exchange, and desalting programs for HisTrap, HiTrap Heparin, and HiPrep Desalting, respectively. Finally, the purified protein was concentrated by ultrafiltration using Amicon Ultra-4 30K device (Millipore). After concentration, the concentration of VTopoI was adjusted to 200 µM and stored at 4°C. The molar concentration of VTopoI was determined by measuring the absorbance of each protein at 280 nm using the molar absorption coefficient of 280 nM calculated by CLC Main workbench (Qiagen, Hilden, Germany).

For T4 PNK, the protein concentration was adjusted to 2 mg/ml (quantified by the Bradford assay using BSA as standard), then an equivalent volume of glycerol was added, and stored at -20°C.

### Formation and purification of VTopoI-ODN complex

Information on the oligonucleotides (ODNs) used in this example is summarized in Table 1.

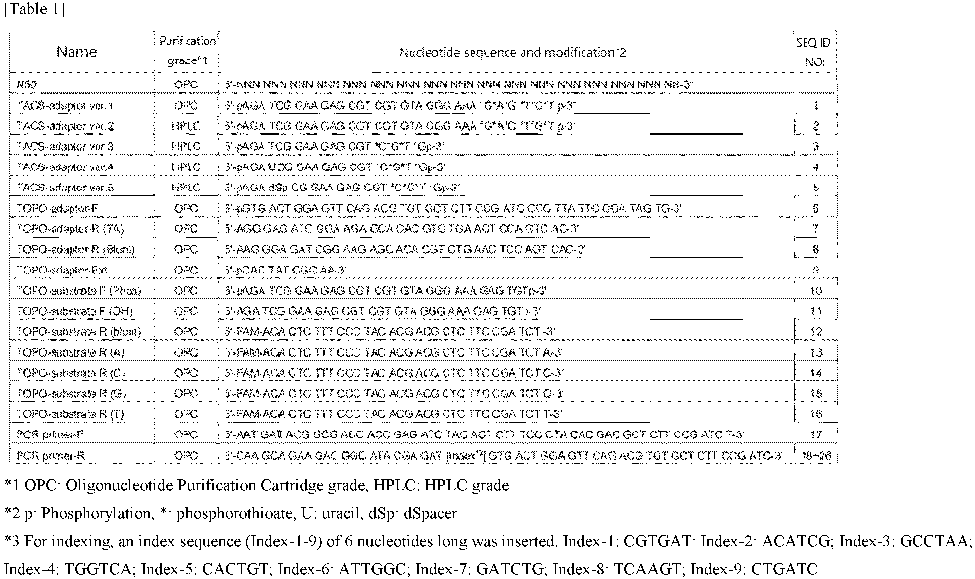

These ODNs were supplied by Eurofin Genomics as OPC- or HPLC-grade purified samples. 1 mL of a reaction solution containing 10 mM Tris-HCl, pH 8.0, 160 mM sodium chloride, 10 µM TOPO-adaptor-F, 10 µM TOPO-adaptor-R (for TA or blunt) and 10 µM TOPO-adaptor-Ext was prepared in a 1.5 ml test tube. This reaction solution was incubated at 95°C for 3 minutes and 55°C for 5 minutes, then maintained at 37°C. Next, in addition to 1 ml of this oligo reaction solution, 1 ml of 5× Topo activation buffer (10 mM Tris-Acetate, pH 8.0, 250 mM potassium acetate, and 50 mM magnesium acetate), 100 µL of 100 mM ATP, and 100 µL of 1 mg/ml T4 PNK were added to a 15 mL centrifuge tube, adjusting the total volume to 5 mL. After incubation at 37°C for 1 hour, the reaction solution was cooled to 30°C and then 250 µL of 200 µM VTopoI was added. This reaction solution was incubated at 30°C for 30 minutes. The reaction solution was then loaded into 1 ml of HiTrap Heparin HP and separated using heparin buffers A and B. Each fraction was analyzed by both SDS-PAGE and agarose gel electrophoresis. For SDS-PAGE, the fractions were separated on Mini-PROTEAN anyKd gel (Bio-rad laboratories) and stained with Bullet CBB stain (Nacalai Tesque, Inc.). For agarose gel electrophoresis, 1 µL of 10% (w/v) SDS solution was added to 10 µL of each fraction, then 1 µl was loaded onto E-Gel Ex (2%) gel for analysis. Two major peaks were confirmed on the fractions in the heparin column, with the fractions containing the VTopoI-ODN complex eluting earlier than VTopoI itself. Fractions containing the VTopoI-ODN complex were collected and their concentrations were adjusted to 150 ng/ µL by ultrafiltration while measuring the concentrations using Qubit dsDNA HS kit. If necessary, the fractions were concentrated using Amicon Ultra-4 30 K device and stored at 4°C until use.

### Isolation of cell-free DNA from human serum

Pooled human sera were purchased from Kohjin Bio Co., Ltd. To an Eppendorf tube, 100 µL of human serum, 400 µL of 10 mM Tris-HCl, pH 8.0, 1.5 µL of 5 M sodium chloride, 10 µL of 10% (w/v) SDS, and 20 µL of Proteinase K (Qiagen) were added and incubated at 50°C for 1 hour. After protease digestion, 500 µL of phenol-chloroform-isoamyl alcohol (25:24:1) was added, stirred vigorously and centrifuged at 15,000 × g for 5 minutes. After centrifugation, 500 µL of the supernatant was taken in a new tube, added and mixed with 50 µL of 3 M sodium acetate (pH 5.4) and 1,000 µL of isopropanol, and centrifuged at 15,000 × g for 5 minutes. After the supernatant was removed by decantation, the DNA pellets were rinsed with 70% (v/v) ethanol and dissolved in an appropriate volume of 10 mM Tris-HCl, pH 8.0. The volume of serum used was changed according to the purpose of the experiment. Even in such cases, the mixing ratio of the reagents used was kept constant.

### -TACS-T4 scheme

Preparation of a library from cfDNA by the TACS-T4 scheme was performed as described in Reference 6.

### -TACS-TOPO scheme

### i) TACS ligation

After adding 2.5 µL of 10× TACS buffer (500 mM HEPES-KOH, pH 7.5, 50 mM MgCl₂, 5% (v/v) Triton X-100) and 1 µL of rSAP (New England Biolab, Ipswich, MA) to a solution containing ssDNA of interest, the volume was adjusted to 11 µL with water. This reaction solution was incubated at 37°C for 15 minutes and 95°C for 5 minutes. Next, in addition to 10 µL of 50% (w/v) PEG6000, 1 µL each of 10 mM ATP, 25 µM adaptor (Table 1), TdT (Takara Bio), and 2 mg/ml TS2126 RNA ligase were added. TS2126 RNA ligase was prepared as described in Reference 6. Note that TS2126 RNA ligase can be substituted with CircLigase II ssDNA ligase supplied by Lucigen. The reaction solution was incubated at 37°C for 15 minutes, 65°C for 30 minutes, and 95°C for 5 minutes.

### ii) Replication of adaptor-ligated ssDNA

To a solution of ssDNA that has been ligated to the adaptor, 14 µL of water, 5 µL of 10× PCR buffer (Nippon Gene Co., Ltd.), 4 µL of 2.5 mM dNTPs, 1 µL of P-PEA2-2T (Table 1), and 1 µL of 5 units/µL Hotstart GeneTaq (Nippon Gene Co., Ltd.) were added. Next, incubations were performed at 95°C for 3 minutes, 45°C for 5 minutes, 55°C for 5 minutes, and 65°C for 5 minutes.

### iii) Second adaptor ligation using VOC

To the reaction solution in which the complementary strand synthesis was completed, 5 µL of 500 mM EDTA (Nacalai Tesque, Inc.) and 1 µL of VOC (for blunt or TA) were added. The reaction was performed at 25°C for 15 minutes.

### iv) Purification of library

After the second adaptor ligation, 45 µL of buffer B2 (3 M guanidine hydrochloride, 20% (v/v) Tween 20) and 5 µL of Proteinase K (Qiagen) were added to the reaction solution and incubated at 50°C for 15 minutes. Next, 2 µL of Sera-Mag Carboxylate Magnetic Beads (Cytiva, Marlborough, MA) and 100 µL of isopropyl alcohol were added and mixed. After 5 minutes of incubation at room temperature, the magnetic particles were captured on a magnetic stand and the supernatant was removed. The beads were then washed twice with 200 µL of bead wash solution (32% (v/v) PEG400, 1 M sodium chloride, 50 mM Tris-HCl, pH 8.0). After rinsing the beads with 70% (v/v) ethanol, the purified DNA was eluted with 25 µL of 10 mM Tris-Acetate, pH 8.0.

### v) UDG-APE1 treatment nick repair, and PCR amplification

To the purified library, 5 µL of 10× ExTaq buffer, 4 µL of 2.5 mM dNTPs, 1 µL of 20 µM primer, 1 µL of UDG (New England Biolabs), and 1 µL of APE 1 (New England Biolabs) were added and the mixture was made up to 50 µL with water. Note that UDG was used only for the version 4 protocol. (See "3. Results" below). The reaction solution was incubated at 37°C for 15 minutes, followed by incubation at 72°C for 5 minutes and 95°C for 1 minute. Then, a three-step incubation process at 95°C for 15 seconds, 55°C for 30 seconds, and 72°C for 1 minute was repeated as many times as necessary. This number of cycles is shown with the data. If necessary, DNA after the reaction was purified using 90 µL of AxyPrep Mag PCR clean. In this case, 21 µL of 10 mM Tris-acetate, pH 8.0 was used for the final DNA eluate.

### -DNA derived from ancient human bones

The ancient human DNA used in this example was provided by Hideaki Kanzawa and Kenichi Shinoda of the National Museum of Nature and Science.

### -Library preparation using commercially available kit

ThruPlex DNA Seq kit (Takara Bio Inc.) was used to prepare a sequencing library targeting double-stranded DNA based on a conventional method. In short, after DNA end repair and adaptor ligation, 50 µL of PCR reaction solution was prepared according to the manual. This reaction tube was incubated at 72°C for 3 minutes, 84°C for 2 minutes, and 98°C for 2 minutes. This was followed by four cycles of a three-step process at 98° for 20 seconds, 67°C for 20 seconds, and 72°C for 40 seconds, and eight cycles of a two-step process at 98°C for 20 seconds and 72°C for 50 seconds. After the reaction, 50 µL of AxyPrep MAG PCR was added to the reaction solution and incubated at room temperature for 5 minutes. After rinsing the magnetic beads with 70% ethanol, the purified DNA was eluted with 25 µL of 10 mM Tris-Acetate, pH 8.0. The purified library was stored at -20°C until use.

### -Library quantification and sequencing

The molar concentration of the sequencing library was determined using Library Quantification Kit from Takara Bio Inc. Small-scale sequencing was performed using MiSeq from Illumina with MiSeq Reagent Kit v3 (150 cycles). Large-scale sequencing was performed by Macrogen, Inc. using HiSeq X Ten from Illumina.

### -cfDNA sequencing

The analysis of cfDNA was performed according to the description in Reference 6.

### -Ancient human DNA sequencing

The obtained reads were first trimmed using fastp [Reference 20] and paired-end reads were merged. The merged reads were then mapped to the human reference genome sequence using bwa [Reference 21] with the mem option. The final output file in the sam format was analyzed using mapdamage [References 22 and 23]. The details of these analyses are as follows

The read sequence data were trimmed and merged by fastp using the following options, and the merged reads were mapped to the human reference genome hg19 using bwa with the "mem" option. Finally, the analysis was performed using mapdamage.

Library prepared using ThruPlex:
--adaptor_sequence = AGATCGGAAGAGCACACGTCTGAACTCCAGTCAC (SEQ ID NO: 29)
--adaptor_sequence_r2 =
   AGATCGGAAGAGCGTCGTGTAGGGAAAGAGTGT (SEQ ID NO: 30)
--merge

Library prepared using TACS-TOPO:
-f 2
-F 4
--adaptor_sequence =
   AAGGGGATCGGAAGAGCACACGTCTGAACTCCAGTCAC (SEQ ID NO: 31)
--adaptor_sequence_r2 =
   AAAGATCGGAAGAGCGTCGTGTAGGGAAAGAGTGT (SEQ ID NO: 32)
--merge

### 3. Results

### -Purification of active VTopoI

Gene of VTopoI was synthesized by artificial gene synthesis and incorporated into three different expression vectors. pColdTF, pColdI and pET28a were used to express VTopoI in bacterial cells. Protein expression was induced and found to be well induced by each vector (Figure 7C). Then, purification with nickel resin using a HisTrap HP column was performed (Figure 7B). Eluted proteins from the nickel columns were further purified with heparin resin (Figure 7C). The purities of the purified proteins were estimated to be 95% or more for all expression vector systems (Figure 7D). The presence or absence of activity of the purified protein was confirmed by the supercoil unwinding assay [Reference 7]. Activity was confirmed for all purified recombinant proteins (Figure 7E). Therefore, the present inventor concluded that VTopoI was successfully purified. For pET28a, the protein yield was found to be lower than the others, so VTopoI produced with pColdI and pColdTF were used in subsequent experiments.

### -Formation of VTopoI-ODN complex and its purification and utilization

VTopoI specifically recognizes a sequence of 5 base pairs 5'-(C/T)CCTT-3' on double-stranded DNA and forms a covalent bond with the 3'-phosphate of said sequence while cleaving the 3' side of said sequence of the substrate DNA [References 9 and 17]. This single-strand cleavage will cause the supercoil to relax. After the supercoiling of the substrate DNA is relaxed, reverse reaction occurs and the cleaved strands rejoin. However, if the substrate DNA has a nick or gap in the complementary strand of the cleavage site, the DNA downstream of the recognition sequence is released from the intermediate complex of the substrate DNA and VTopoI, resulting in an active VTopoI-DNA complex (Figure 1A, reaction 1). If DNA with a suitable end is present in the vicinity of this complex, VTopoI causes reverse reaction and joins with this DNA [Reference 17]. This reaction is fast and highly efficient and is the basis of the ligation reaction by Topo. The reaction between the substrate DNA containing 5'-(C/T)CCTT-3'- and VTopoI is bidirectional, and the ratio of free VTopoI to VTopoI-DNA complex is determined by the concentration of these molecules (Figure 1A, reaction 1). To increase the amount of the intermediate formed by the covalent bonding between VtopoI and DNA, it would be effective to inhibit the reaction in the reverse direction (Figure 1A, reaction 2). This can be achieved by phosphorylating the 5'-end of the DNA released upon complex formation. (Figure 1A, reaction 2). This is because once the 5'-end of the released DNA is phosphorylated, it cannot participate in the reverse reaction. As expected, the amount of the intermediate (VOC) formed by the covalent bond between VTopoI and ODN increased by adding ATP and T4 PNK to the reaction solution (Figures 1A-1C). The composition of the reaction solution also affects the amount of VOC formed. The presence of acetate ions has been found to be more effective in complex formation than chloride ions due to a poorly understood mechanism (Figures 1B and 1C). The present inventor also noticed that VTopoI produced with pColdI was more likely to form precipitates during the formation of VOC (data not shown). Therefore, VTopoI produced with pColdTF was used for subsequent development. Based on these results and other studies, more effective conditions for VOC formation were selected.

After VOC formation, the reaction solution contains unreacted ODN, VTopoI, and T4 PNK. Since these molecules cause side reactions in downstream operations, they need to be removed in advance. Again, purification using a heparin column proved effective (Figure 1D). Unreacted ODN is negatively charged and therefore cannot bind to the negatively charged heparin column. VOC can bind to the heparin column via VTopoI and was found to elute at a lower salt concentration than VTopoI itself or T4 PNK (Figure 1D). Therefore, it was found that the isolation of VOC from other unreacted components could be achieved by only one chromatographic run using a heparin column. After this purification, VOC can be easily concentrated by ultrafiltration. The purified VOC showed sufficiently high ligation activity with model double-stranded DNA (see below), and this activity was not lost during storage at 4°C for several months.

### -VOC ligates adaptor to double-stranded DNA with unphosphorylated 5'-end with high efficiency

The present inventor then decided to study the specificity and efficiency of ligation by VOC. Here, double-stranded ODN was prepared as a model substrate for the experiment (TOPO-substrate). This TOPO-substrate accepts ligation by VOC at one end (active end), while the reaction is blocked at the other end (inactive end) by 5'-FAM and 3'-phosphorylation (Figure 2A). To study the stringency of the ligation reaction by VOC with respect to the end nucleotide, TOPO-substrates with a blunt end or an A, C, G, or T single-base 3' overhang at the active end were prepared. In order to study the specificity of VOC with respect to the phosphorylated state of the substrate, TOPO-substrates with 5'-hydroxylated or 5'-phosphorylated active ends were prepared. Two types of VOCs, namely blunt and TA-ligated types, were prepared (Figure 2A). The present inventor studied the ligation efficiency for all of these TOPO-substrate and VOC combinations (Figure 2B).

High ligation activity to TOPO-substrates with compatible end structures was confirmed for both blunt- and TA-type VOCs. This reaction almost completed in just a few minutes in the presence of a sufficient amount of VOC (Figure 2B). VOC showed strong activity for the 5'-hydroxylated end, whereas it never reacted with those with 5'-phosphorylated end (Figure 2B). Although ligation of the adaptor to a TOPO-substrate with a compatible end was efficient, the stringency of base pairing with the end structure was not as high as that with the phosphorylated end. The TA-type VOC showed ligation activity to TOPO-substrates with a T-, C-, or G-overhang, while the blunt type reacted with TOPO-substrates with any overhanging ends. It has been shown that the stringency of the compatibility of the end structure can be enhanced by the addition of sodium chloride [Reference 24], but increasing the stringency resulted in a decrease in ligation efficiency (data not shown).

The property of VOC to be specific for 5'-unphosphorylated substrates is ideal for the second adaptor ligation after the TACS ligation (see summary in section 1 above). Therefore, the present inventor proceeded to develop a new library preparation protocol using VOC.

### -TACS-TOPO scheme can suppress adaptor dimer formation

Since VOC showed efficient adaptor ligation to the 5'-unphosphorylated end and did not react with the 5'-phosphorylated end (Figure 2C), the present inventor thought that VOC might be able to realize efficient second adaptor ligation without causing adaptor dimer formation. When 83 ng (5 pmol) of random 50 nucleotides (N50) were used as a model, the yield of the final product of interest ligated with two adaptors by the TACS-T4 scheme was low (Figure 3A, lane 4, green star), while the TACS-TOPO scheme showed a dramatic improvement in library yield (Figure 3B, lane 4, green star). In addition, analysis of the PCR-amplified library showed that a large amount of adaptor dimers was contained by the TACS-T4 scheme, while only library molecules of the desired size, containing the inserts, were amplified by the TACS-TOPO scheme (Figure 3C). These results showed that the TACS-TOPO scheme was superior to the TACS-T4 scheme both in terms of higher library yield and less adaptor dimer formation.

### -Optimization of TACS-TOPO scheme to realize highly sensitive dimer-free library preparation

Next, the present inventor studied the sensitivity of the TACS-TOPO scheme to prepare an adaptor dimer-free library. N50 was again used as a model to study the formation of adaptor dimers. As shown in Figure 4B, formation of a non-negligible amount of adaptor dimers was observed even by the TACS-TOPO scheme when the amount of input DNA was lower than 24 ng. This phenomenon appears to be caused in part by a very small amount of molecules that lost the phosphorylation of 5'-ends contained in the adaptor ODNs. This was suggested from the finding that the amount of adaptor dimers formed was reduced by simply increasing the purification grade of the adaptor ODNs (version 2, Figure 4C and Figures 8A-8D). Unfortunately, attempts to suppress adaptor dimer formation through additional purification of the adaptor ODNs were unsuccessful (data not shown), and thus the present inventor decided to suppress adaptor dimer formation by combining two other experimental principles. The first strategy was to reduce the size of the adaptor molecule itself to facilitate the removal of the adaptor dimer (version 3, Figures 8A-8E). The other was to insert uracil at a site near the 5' end of the adaptor ODN to degrade the adaptor dimer before PCR amplification (version 4, Figures 8A, 8B, and 8F). By combining these two tricks with the TACS-TOPO scheme, it was possible to prepare a library with less dimer formation from as little as 24 pg of DNA, which is equivalent to 4 human cells. In other words, TACS-TOPO version 4 has made it possible to prepare an adaptor dimer-free library from a very small amount of single-stranded DNA.

### -Library preparation from short single-stranded cell-free DNA in peripheral blood

Previously, the present inventor discovered that single-stranded cell-free DNA of approximately 50 nucleotides was found in human peripheral blood, and named it C3D [Reference 6]. The present inventor analyzed the cfDNA using the TACS-T4 scheme and found that C3D is a new class of cfDNA. However, the TACS-T4 scheme predominantly amplified adaptor dimers, which required repeated laborious SPRI purification to remove them. Since the optimized TACS-TOPO scheme is effective for preparing a dimer-free library from a limited amount of single-stranded DNA (Figures 4C and 4D), the present inventor decided to apply TACS-TOPO version 4 to library preparation from cfDNA. As shown in Figure 5A, while the preparation of a adaptor dimer-free sequencing library was realized with TACS-TOPO version 4, the amount of adaptor dimers was predominant with TACS-T4, despite the purification step with SPRI. As previously shown [Reference 6], two large peaks corresponding to nucleosome-size cfDNA (160 nucleotide long insert) and C3D were shown to be present in the PCR-amplified library, indicating that TACS-TOPO version 4 is useful for the analysis of cfDNA. A sufficient amount of library was prepared from cfDNA purified from 50 µL of serum without amplifying the entire library by PCR, and the required amount of library was obtained even from 6.25 µL of serum with 3 cycles (up to 8×) of PCR amplification (Figures 5B and 5C). Sequencing of the resulting library and analysis of its reads identified the characteristics of C3D previously shown (Figures 5D and 5E) [Reference 6]. These results demonstrated the practical utility of the TACS-TOPO scheme in the analysis of cfDNA.

### -TACS-TOPO scheme realizes highly sensitive library preparation from DNA derived from ancient organism

The TACS-TOPO scheme was applied to library preparation from ancient samples. In many cases, DNA derived from ancient samples has been highly degraded, manifesting as fragmentation and conversion into single-stranded DNA. Therefore, a method for preparing a library from such samples should be able to work with single-stranded DNA. In fact, library preparation methods that work with single-stranded DNA have been shown to be effective for the analysis of ancient DNA by Gansauge et al. [References 2 and 3]. The TACS-T4 scheme was applied to cfDNA and resulted in a significant improvement in library yield compared to the scheme established by Gansauge et al. [Reference 6], and the TACS-TOPO scheme performed even better than the TACS-T4 scheme in the analysis of cfDNA (Figure 5A). Therefore, the TACS-TOPO scheme was considered more suitable for preparing libraries from ancient samples.

The present inventor decided to use DNA extracted from human bones excavated from the Inome and Shimekake sites for this verification. Each site is from the late Jomon period, so the DNA obtained is derived from humans 2,000-4,000 years old and is naturally expected to be highly fragmented and to contain many obstacles. The yield of library preparation from these ancient samples with TACS-TOPO version 4 was extremely good compared to ThruPlex DNA-Seq kit, one of the major commercial library kits adapted for library preparation from double-stranded DNA. Specifically, library yield improved by one to two orders of magnitude by the TACS-TOPO scheme (Figure 6A). Meanwhile, there was a clear difference in the size distribution of the libraries prepared by the ThruPlex and TACS-TOPO schemes. The latter library always showed a shorter size distribution than the former (Figures 6B and 6C). In addition, the mapping rates to the human reference genome sequence were similar between the reads obtained from the libraries prepared by the ThruPlex and TACS-TOPO schemes (Figure 6D).

The yields of sequencing libraries derived from ancient human bones improved dramatically with TACS-TOPO version 4. However, although C to T or G to A nucleotide substitutions should be observed at both ends of the reads, the reads mapped to the human reference genome sequence lacked the mismatch distribution pattern characteristic of this ancient DNA at one end (Figure 6F). This may be due to the fact that the UDG-APE1 treatment is incorporated in TACS-TOPO version 4 to degrade adaptor dimers, and this enzymatic treatment caused degradation of uracil (U) resulting from deamination of C.

The present inventor sought to use modifications other than uracil for the adaptor used in TACS ligation to avoid the treatment with UDG-APE1. After studying several modifications, the present inventor found that dSpacer (dSp), an analog of the AP site, could be used for this purpose. Since dSp is known to be degraded by APE 1, the present inventor initially added treatment with APE 1 prior to PCR. However, for some unknown reason, treatment with APE1 was found to be unnecessary in practice, and thus this was found to be advantageous for simplicity. The present inventor applied this modified protocol (TACS-TOPO version 5, Figures 8A and 8G) to ancient human DNA. As a result, reads obtained from a library prepared with an adaptor containing dSp contained C to T nucleotide substitutions at both ends of the reads (Figure 6G). Although the library prepared with the dSp-containing adaptor had a slightly lower library yield than that prepared with uracil (Figure 6A), this version 5 protocol had a library size distribution comparable to that obtained with the version 4 protocol (Figure 6B-6D). These results indicate that TACS-TOPO version 5 can be an extremely useful tool in biomolecular archaeology.

The following is a list of the references mentioned in the example.
1. Meyer, M., Kircher, M., Gansauge, M.T., Li, H., Racimo, F., Mallick, S., Schraiber, J.G., Jay, F., Prufer, K., de Filippo, C. et al. (2012) A high-coverage genome sequence from an archaic Denisovan individual. Science, 338, 222-226.
2. Gansauge, M.T. and Meyer, M. (2013) Single-stranded DNA library preparation for the sequencing of ancient or damaged DNA. Nat. Protoc. 8, 737-748.
3. Gansauge, M.T., Gerber, T., Glocke, I., Korlevic, P., Lippik, L., Nagel, S., Riehl, L.M., Schmidt, A. and Meyer, M. (2017) Single-stranded DNA library preparation from highly degraded DNA using T4 DNA ligase. Nucleic Acids Res. 45, e79.
4. Miura, F., Shibata, Y., Miura, M., Sangatsuda, Y., Hisano, O., Araki, H. and Ito, T. (2019) Highly efficient single-stranded DNA ligation technique improves low-input whole-genome bisulfite sequencing by post-bisulfite adaptor tagging. Nucleic Acids Res. 47, e85.
5. Schmidt, W.M. and Mueller, M.W. (1996) Controlled ribonucleotide tailing of cDNA ends (CRTC) by terminal deoxynucleotidyl transferase: a new approach in PCR-mediated analysis of mRNA sequences. Nucleic Acids Res., 24, 1789-1791.
6. Hisano, O., Ito, T. and Miura, F. (2021) Short single-stranded DNAs with putative non-canonical structures comprise a new class of plasma cell-free DNA. BMC Biol. 19, 225.
7. Shuman, S. and Moss, B. (1987) Identification of a vaccinia virus gene encoding a type I DNA topoisomerase. Proceedings of the National Academy of Sciences, 84, 7478-7482.
8. Shuman, S., Golder, M. and Moss, B. (1988) Characterization of vaccinia virus DNA topoisomerase I expressed in Escherichia coli. J. Biol. Chem., 263, 16401-16407.
9. Shuman, S. and Prescott, J. (1990) Specific DNA cleavage and binding by vaccinia virus DNA topoisomerase I. J. Biol. Chem., 265, 17826-17836.
10. Shuman, S. (1991) Recombination mediated by vaccinia virus DNA topoisomerase I in Escherichia coli is sequence specific. Proc. Natl. Acad. Sci. U. S. A., 88, 10104-10108.
11. Shuman, S. (1991) Site-specific interaction of vaccinia virus topoisomerase I with duplex DNA. Minimal DNA substrate for strand cleavage in vitro. J. Biol. Chem., 266, 11372-11379.
12. Shuman, S. (1991) Site-specific DNA cleavage by vaccinia virus DNA topoisomerase I. Role of nucleotide sequence and DNA secondary structure. J. Biol. Chem., 266, 1796-1803.
13. Shuman, S. (1992) Two classes of DNA end-joining reactions catalyzed by vaccinia topoisomerase I. J. Biol. Chem., 267, 16755-16758.
14. Morham, S.G. and Shuman, S. (1992) Covalent and noncovalent DNA binding by mutants of vaccinia DNA topoisomerase I. J. Biol. Chem., 267, 15984-15992.
15. Shuman, S. (1992) DNA strand transfer reactions catalyzed by vaccinia topoisomerase I. J. Biol. Chem., 267, 8620-8627.
16. Stivers, J.T., Shuman, S. and Mildvan, A.S. (1994) Vaccinia DNA topoisomerase I: kinetic evidence for general acid-base catalysis and a conformational step. Biochemistry, 33, 15449-15458.
17. Shuman, S. (1994) Novel approach to molecular cloning and polynucleotide synthesis using vaccinia DNA topoisomerase. J. Biol. Chem., 269, 32678-32684.
18. Bauer, W.R., Ressner, E.C., Kates, J. and Patzke, J.V. (1977) A DNA nicking-closing enzyme encapsidated in vaccinia virus: partial purification and properties. Proc. Natl. Acad. Sci. U. S. A., 74, 1841-1845.
19. Yarovinsky, T.O. (2000) Application of DNA topoisomerase-activated adapters to riboprobe synthesis. Biotechniques, 28, 1160, 1162-1165.
20. Chen, S., Zhou, Y., Chen, Y. and Gu, J. (2018) fastp: an ultra-fast all-in-one FASTQ preprocessor. Bioinformatics, 34, i884-i890.
21. Li, H. and Durbin, R. (2009) Fast and accurate short read alignment with Burrows-Wheeler transform. Bioinformatics, 25, 1754-1760.
22. Ginolhac, A., Rasmussen, M., Gilbert, M.T.P., Willerslev, E. and Orlando, L. (2011) mapDamage: testing for damage patterns in ancient DNA sequences. Bioinformatics, 27, 2153-2155.
23. Jónsson, H., Ginolhac, A., Schubert, M., Johnson, P.L.F. and Orlando, L. (2013) mapDamage2.0: fast approximate Bayesian estimates of ancient DNA damage parameters. Bioinformatics, 29, 1682-1684.
24. Cheng, C. and Shuman, S. (2000) DNA strand transfer catalyzed by vaccinia topoisomerase: ligation of DNAs containing a 3' mononucleotide overhang. Nucleic Acids Res., 28, 1893-1898.

### INDUSTRIAL APPLICABILITY

The present invention provides a method for preparing a double-stranded DNA library from single-stranded DNA fragments, which can increase the library yield extremely efficiently compared to conventional double-stranded DNA library preparation methods and which can even effectively suppress the production of double-stranded adaptor dimers as by-products during the preparation.

## Claims

1. A method for preparing a double-stranded DNA library from single-stranded DNA fragments, comprising:
(i) a step of ligating a single-stranded adaptor sequence A1 having a phosphorylated 5'-end to the 3'-end of a single-stranded DNA fragment having both ends dephosphorylated to produce adaptor-tagged single-stranded DNA;
(ii) a step of annealing a single-stranded adaptor sequence A2 to the adaptor sequence A1 portion contained in the adaptor-tagged single-stranded DNA and then elongating the 3'-end of the adaptor sequence A2 by DNA polymerase to produce adaptor-tagged double-stranded DNA;
(iii) a step of reacting topoisomerase with a double-stranded adaptor sequence B1, which has only one 5'-end phosphorylated and has a topoisomerase recognition sequence in the region containing the 3'-end complementary to said 5'-end, to produce a topoisomerase-linked double-stranded adaptor sequence B2 having topoisomerase conjugated to the 3'-end of said recognition sequence;
(iv) a step of ligating the topoisomerase-linked double-stranded adaptor sequence B2 to the end of the adaptor-tagged double-stranded DNA, which end is tagged with no adaptor sequence, to produce double-stranded DNA having both ends tagged with adaptors; and
(v) a step of performing PCR using the double-stranded DNA having both ends tagged with adaptor sequences as a template to obtain a library containing a plurality of said double-stranded DNAs.

2. The method according to claim 1, wherein the topoisomerase-linked double-stranded adaptor sequence B2 is not ligated to a double-stranded adaptor sequence C produced by annealing between unreacted single-stranded adaptor sequence A1 and unreacted single-stranded adaptor sequence A2.

3. The method according to claim 1, wherein:
the double-stranded adaptor sequence B1 has a nick in the region containing the phosphorylated 5'-end; and
the single-stranded adaptor sequence A1 has uracil or dSpacer in the sequence.

4. The method according to claim 3, wherein the step (iv) comprises adding endonuclease to the reaction system after the production.

5. The method according to claim 1, wherein the ligation and production in step (i) are performed using the TACS ligation scheme.

6. A double-stranded DNA library obtained by the method according to any one of claims 1-5.
